# EUROPEAN PATENT APPLICATION

(11) **EP 4 545 076 A1**
(43) Date of publication of application: **30.04.2025**
(21) Application number: 23383082.7
(22) Date of filing: 24.10.2023
(51) Int. Cl.: A61K 31/185, A61K 31/19, A61K 31/198, A61K 31/403, A61K 31/423, A61K 31/4353, A61P 9/00

(54) **LAT1 SELECTIVE INHIBITORS FOR USE IN THE TREATMENT OF ADVERSE VENTRICULAR -REMODELLING AND OF HEART FAILURE**

(71) Applicant: Fundació Hospital Universitari Vall d'Hebron - Institut de Recerca, 08035 Barcelona (ES)
(72) Inventor: INSERTE IGUAL, Javier, 08107 MARTORELLES (ES); DELGADO TOMÀS, Sara, 08035 BARCELONA (ES); FERREIRA GONZÁLEZ, Ignacio, 08005 BARCELONA (ES); ALUJA GONZÁLEZ, David, 08025 BARCELONA (ES); BARRABÉS RIU, José Antonio, 08035 BARCELONA (ES)
(74) Representative: ZBM Patents - Zea, Barlocci & Markvardsen

(57) **Abstract**

The present disclosure relates to LAT1 selective inhibitors for use in the prophylactic or the therapeutic treatment of adverse ventricular remodelling or of heart failure. It also relates to drug combinations of a LAT1 inhibitor and other drugs addressed to the treatment of adverse ventricular remodelling and of heart failure, as well as to pharmaceutical compositions and kits containing them, and to the use of the latter in for any one of the mentioned treatments.

## Description

### TECHNICAL FIELD

The present disclosure relates to the field of medical approaches for cardiopathies. In particular to the field of adverse ventricular remodelling and of heart failure in which a sustained stress of the myocardium takes place.

### BACKGROUND ART

Heart failure is a complex and heterogeneous syndrome with a major socioeconomic impact. It is a leading cause of death and hospitalisation, with very negative consequences on the quality of life of patients and caregivers. Its burden, despite all the advances in its treatment, increases with the ageing of the population.

Heart failure is invariably preceded by ventricular remodelling characterised by hypertrophy and changes in the extracellular matrix with increased fibrosis.

Ventricular remodelling results as a response to myocardial stress accompanying many cardiopathies, such as ischemia, arterial hypertension, valve diseases, myocarditis, and dilated cardiomyopathy. This initially compensatory process evolves to adverse ventricular remodelling if stress persists. As a consequence there is cardiomyocyte hypertrophy, proinflammatory cell infiltration, and excessive collagen deposition in interstitial matrix of myocardium, causing functional impairment and chronic heart failure.

Nowadays, there is not any specific treatment for the adverse ventricular remodelling. Pharmacological treatment of heart failure is based on modulation of the renin-angiotensin-aldosterone system and the sympathetic system with angiotensin-converting enzyme (ACE) inhibitors, angiotensin receptor neprilysin inhibitors (ARNI), beta-adrenergic receptor inhibitors, or mineralocorticoid receptor antagonists, and more recently, with sodium-glucose cotransporter type 2 (SGLT2) inhibitors. Despite the full battery of drugs targeting this heterogeneous patient population, their effectiveness is limited.

Both, hypertrophy and fibrosis are associated with increased protein synthesis and thus also with changes in amino acid (AA) metabolism and increased transport.

L-type amino acid transporter 1 (LAT1, also known as SLC7A5) is a key AA transporter/sensor involved in the uptake of extracellular essential AA such as leucine, which has been shown to promote the activation of mTORC1, the master regulator that couples AA availability to protein synthesis.

LAT1 expression is correlated with cancer malignancy, suggesting that LAT1 is a promising target for cancer therapy.

JPH203 (also known as nanvuranlat or O-[(5-amino-2-phenyl-1,3-benzoxazol-7-yl)methyl]-3,5-dichloro-L-tyrosine; CAS number 1037592-40-7) 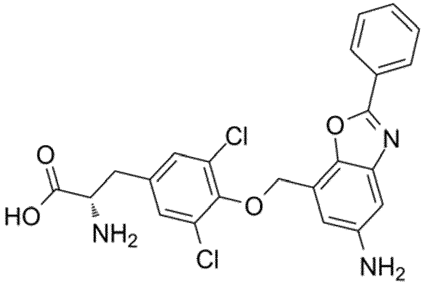 is a LAT1 selective inhibitor, that has been shown to reduce the uptake of neutral amino acids such as leucine and to inhibit cancer cell growth of different cancer cell lines.

As stated above, despite all the advances in the pharmacological treatment of the heart failure population, this heterogeneous syndrome remains a major cause of death and hospitalisation. Therefore, there is an urgent need to find new strategies targeting the alterations that cause cardiac dysfunction, particularly, of a specific effective treatment for ventricular remodelling and/or heart failure.

### SUMMARY OF THE INVENTION

Inventors have surprisingly found that the inhibition of the essential AA transporter LAT1 (slc7a5) reduced the development of cardiac remodelling and its progression to heart failure by reducing cardiac hypertrophy and myocardial fibrosis. Particularly, they have found that prolonged (also termed herein sustained or chronic) administration of a LAT1 selective inhibitor such as JPH203 have positive effect on adverse ventricular remodelling (AVR) and attenuate heart failure caused for example by aortic stenosis. Such inhibitors are also effective for preventing AVR and heart failure induced by other pathologies causing sustained myocardial stress such as myocardial infarct.

As shown in the examples below, pressure overload induces AA metabolic reprogramming characterized by enhanced AA transport and inhibited BCAA catabolism and that essential AA transporter LAT1 (slc7a5) overexpression occurs in response to cardiac stress and contributes to adverse cardiac remodelling and HF.

In particular, as also illustrated in the examples below, the administration of a LAT1 selective inhibitor such as JPH203 attenuates amino acid uptake and reverses the inhibition of branched chain amino acid catabolism induced by aortic stenosis. Additionally, it has been proved that chronic administration of the LAT1 selective inhibitor JPH203 attenuates cardiac hypertrophy, cardiac fibrosis, ventricular dilatation and cardiac dysfunction.

Therefore, the administration of the of a LAT1 selective inhibitor suppose a real advantage for subjects suffering from or at risk of suffering HF, even if this administration is not for once, but for a long period of time.

Thus, a first aspect of the present disclosure relates to a LAT1 selective inhibitor or a pharmaceutically acceptable salt thereof, or any stereoisomer or mixtures of stereoisomers of any of them, for use in the prophylactic or the therapeutic treatment of adverse ventricular remodelling or in the prophylactic or the therapeutic treatment of heart failure.

A second aspect of the present disclosure relates to a pharmaceutical composition comprising a therapeutically effective amount of a LAT1 selective inhibitor or a pharmaceutically acceptable salt thereof as defined herein above or below, or any stereoisomer or mixtures of stereoisomers of any of them, together with one or more pharmaceutically acceptable excipients or carriers, for use in the prophylactic or the therapeutic treatment either of adverse ventricular remodelling or of heart failure.

Apart from the potential use of LAT1 selective inhibitor as monotherapy of either adverse ventricular remodelling or of heart failure, the LAT1 selective inhibitor may be advantageously used in combination with other therapeutic agents known for the same indication such as angiotensin-converting enzyme (ACE) inhibitors, angiotensin receptor neprilysin inhibitors (ARNi), beta-adrenergic receptor inhibitors, mineralocorticoid receptor antagonists, and sodium-glucose cotransporter type 2 (SGLT2) inhibitors.

Thus, a third aspect of the present disclosure relates to a drug combination comprising: a) a LAT1 selective inhibitor or a pharmaceutically acceptable salt thereof, or any stereoisomer or mixtures of stereoisomers of any of them, and b) a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor, all of them as defined herein below.

The drug combinations of the present disclosure may be formulated in different types of compositions or kits of parts for use together.

Thus, a fourth aspect of the present disclosure relates to a single pharmaceutical composition which comprises: a) a therapeutically effective amount of a LAT1 selective inhibitor or a pharmaceutically acceptable salt thereof, or any stereoisomer or mixtures of stereoisomers of any of them; b) a therapeutically effective amount of a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor as defined herein; and one or more pharmaceutically acceptable excipients or carriers.

A fifth aspect of the present disclosure relates to a kit of parts comprising: a) a first pharmaceutical composition which comprises a therapeutically effective amount of a LAT1 selective inhibitor or a pharmaceutically acceptable salt thereof, or any stereoisomer or mixtures of stereoisomers of any of them, together with one or more pharmaceutically acceptable excipients or carriers; and a) a second pharmaceutical composition which comprises a therapeutically effective amount of a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor as defined herein, together with one or more pharmaceutically acceptable excipients or carriers; wherein compositions i) and ii) are separate compositions.

Further, as previously indicated, the drug combinations of the present disclosure may be used for the treatment either of adverse ventricular remodelling or of heart failure. Thus, a sixth aspect of the present disclosure relates to the drug combination, the single pharmaceutical composition, or the package or kit of parts as defined herein, for use in the prophylactic or the therapeutic treatment either of adverse ventricular remodelling and/or of heart failure.

A seventh aspect of the present disclosure relates to a LAT1 selective inhibitor or a pharmaceutically acceptable salt thereof, or any stereoisomer or mixtures of stereoisomers of any of them, for use in combination therapy in the prophylactic or the therapeutic treatment either of adverse ventricular remodelling and of heart failure, wherein a LAT1 selective inhibitor or a pharmaceutically acceptable salt thereof, or any stereoisomer or mixtures of stereoisomers of any of them, is to be administered simultaneously, concurrently, separately or sequentially with one or more therapeutic agents selected from the group consisting of angiotensin-converting enzyme (ACE) inhibitors, angiotensin receptor neprilysin inhibitors (ARNi), beta-adrenergic receptor inhibitors, mineralocorticoid receptor antagonists, and sodium-glucose cotransporter type 2 (SGLT2) inhibitors as defined herein.

### BRIEF DESCRIPTION OF THE DRAWINGS

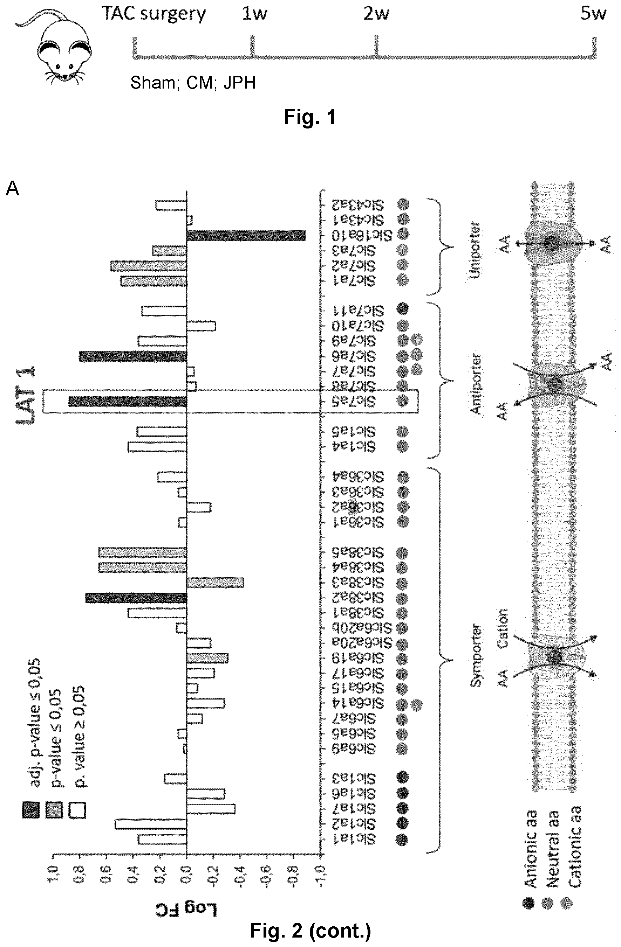

FIG. 1 depicts the experimental protocol that was followed to test the potential of the LAT1 inhibitor (JPH203) for the treatment of adverse ventricular remodelling and heart failure induced by aortic stenosis. Aortic stenosis was produced by transverse aortic constriction (TAC) in C57BL/6 mice from Charles River and mice were euthanised 5 weeks after surgery. C means control (mice without JPH203 administration); JPH means that JPH203 was intraperitoneally administered (bolus 12.5 mg/kg/day in 40% 2-hydroxipropil-beta-cyclodextrin) once daily.

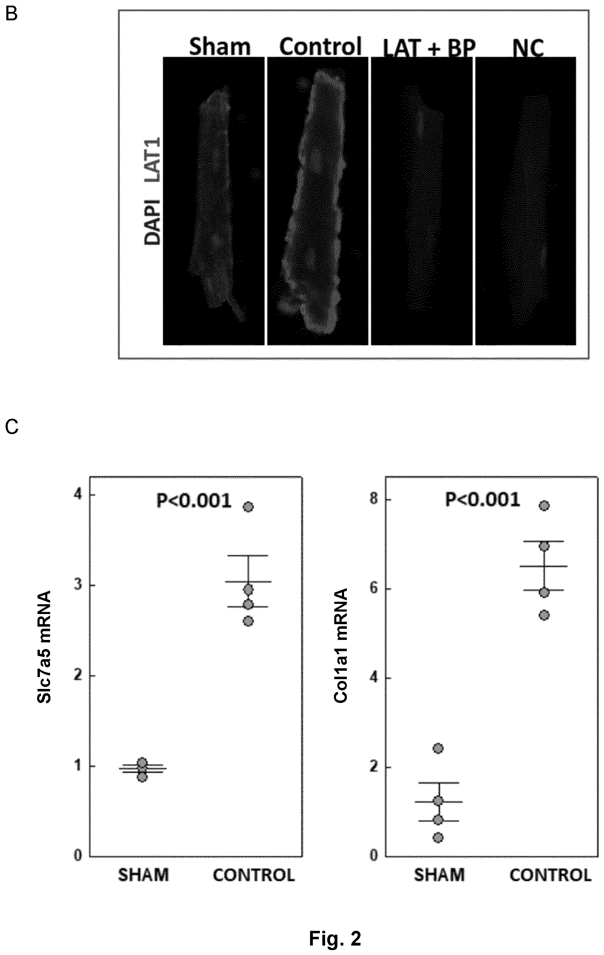

FIG. 2 shows the increase in LAT1 expression induced by TAC. Fig. 2A reflects the changes in the expression of genes expressing proteins involved in the transmembrane transport of amino acids. Data were obtained from a microarray assay performed using myocardial samples obtained from Sham and Control groups (Control vs. Sham depicted). Red, grey and white bars correspond to adjusted p-value<0.05, p-value<0.05 and p-value>0.05 respectively. Fig. 2B shows a confocal image corresponding to a cardiomyocyte isolated from a Sham and Control mice and stained with an antibody against LAT1 and DAPI. DAPI was used to stain DNA. LAT+BP means that the cardiomyocyte was stained with LAT1 antibody and a peptide directed to block this antibody. BP means blocking peptide and NC means negative control. Fig. 2C shows the expression of LAT1 (Slc7a5) and collagen 1 (Col1 a1) measured by RT-PCR using mRNA extracted from fibroblast isolated from Sham and control mice. Data (fold change vs. Sham) are presented as mean±SEM. Groups were compared by using one-way analysis of variance (ANOVA) followed by Tukey HSD test.

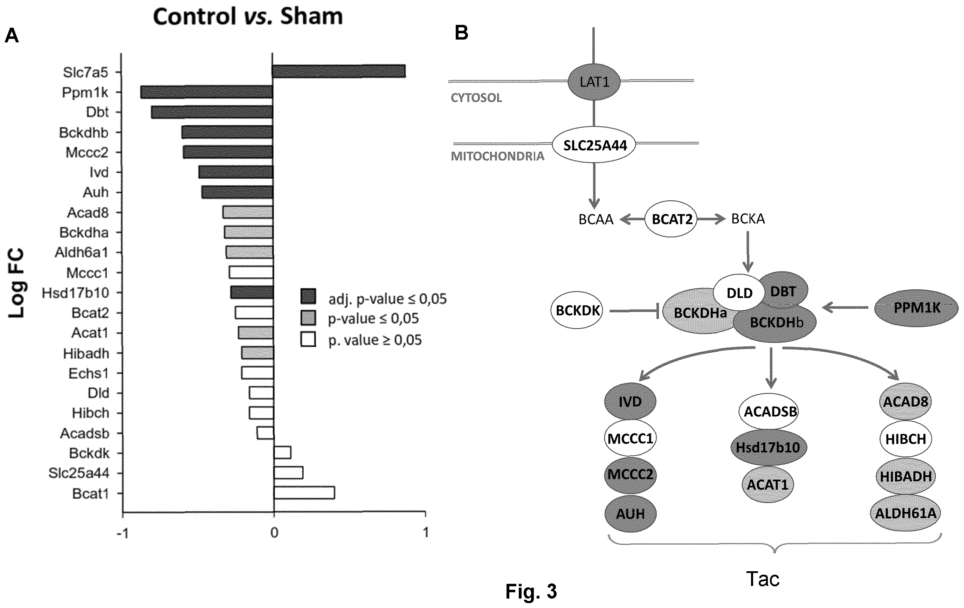

Fig. 3 shows results corresponding to the expression of genes involved in the metabolism of branched chain amino acids (BCAA). Fig. 3A shows the differences in the expression of these genes between Sham and control group. X°Data were obtained from a microarray assay performed using myocardial samples from sham and control mice. Red, grey and white bars correspond to adjusted p-value<0.05, p-value<0.05 and p-value>0.05 respectively. Fig. 3B present an schematic description of the genes involved in BCAA metabolism that are modified by aortic stenosis (control group). Tac means Tricarboxylic acid cycle.

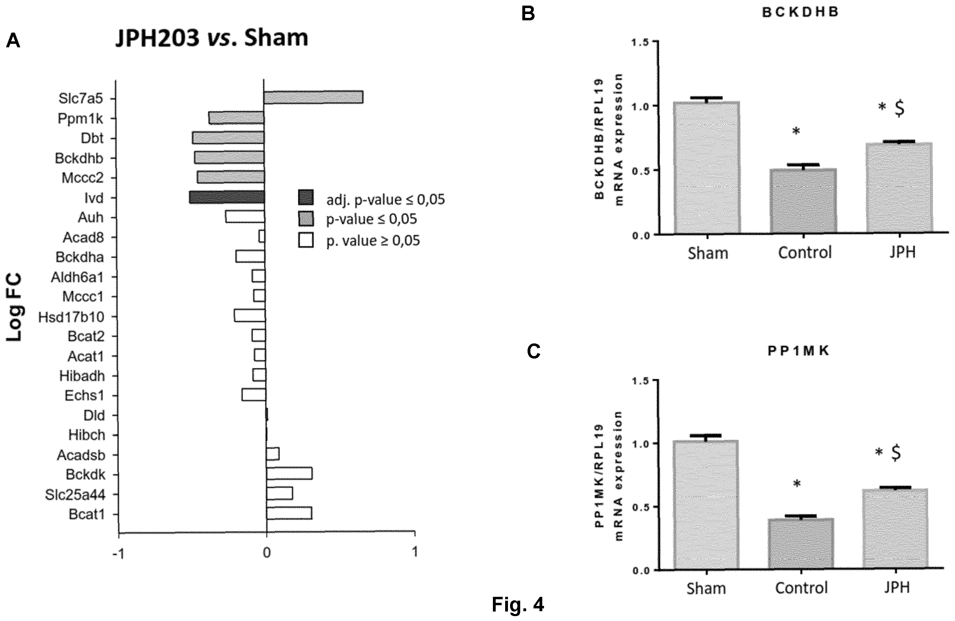

Fig. 4 shows results corresponding to the expression of genes involved in the metabolism of branched chain amino acids (BCAA). Fig. 4A. shows the differences in the expression of these genes between JPH203 treated mice and the sham group. Data were obtained from a microarray assay performed using myocardial samples from sham mice and mice treated with JPH203 as depicted in Fig. 1. Red, grey and white bars correspond to adjusted p-value<0.05, p-value<0.05 and p-value>0.05 respectively. Fig. 4B correspond to the expression of BCKDHB and Fig. 4C correspond to the expression of PP1MK measured by RT-PCR using mRNA extracted from hearts corresponding to the groups depicted in Fig. 1. These two genes express enzymes that are critical for the regulation of BCAA catabolism, as shown in Fig. 3B. Results are mean±SEM (n=5 per group). Groups were compared by using one-way analysis of variance. (ANOVA) followed by Tukey HSD test. *p<0.05 respect to Sham group; ^{$}p<0.05 respect to control group.

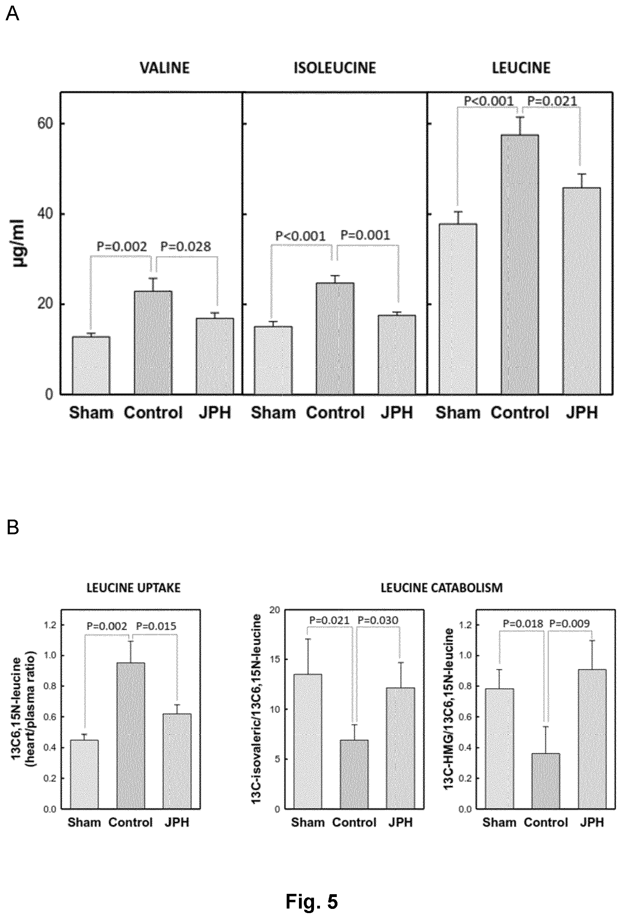

Fig. 5 shows metabolomic data linked to the metabolism of amino acids obtained by LC-MS/MS. Fig. 5A shows the myocardial concentration of the BCAA (valine, isoleucine and leucine) in the groups described in Fig. 1. Fig. 5B correspond to the evaluation of changes in leucine uptake, measured as the concentration of 13C,15N-leucine ratio in heart homogenates and plasma samples, and leucine catabolism, calculated as the concentration in heart homogenates of the leucine-derived catabolites 13C-isovaleric and 13C- β-hydroxy β-methyl butyrate (HMG). Heart homogenates were obtained from mice corresponding to the groups depicted in Fig. 1 and values were corrected by the 13C,15N-leucine. Mice were pre-treated with an intraperitoneal administration of 13C,6N-leucine 15 minutes before euthanasia. Data are presented as mean±SEM. Groups were compared by using one-way analysis of variance. (ANOVA) followed by Tukey HSD test

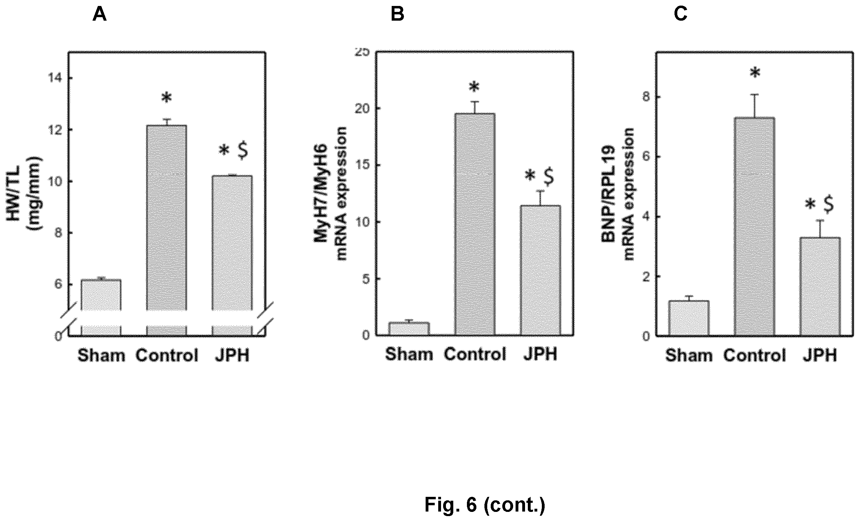

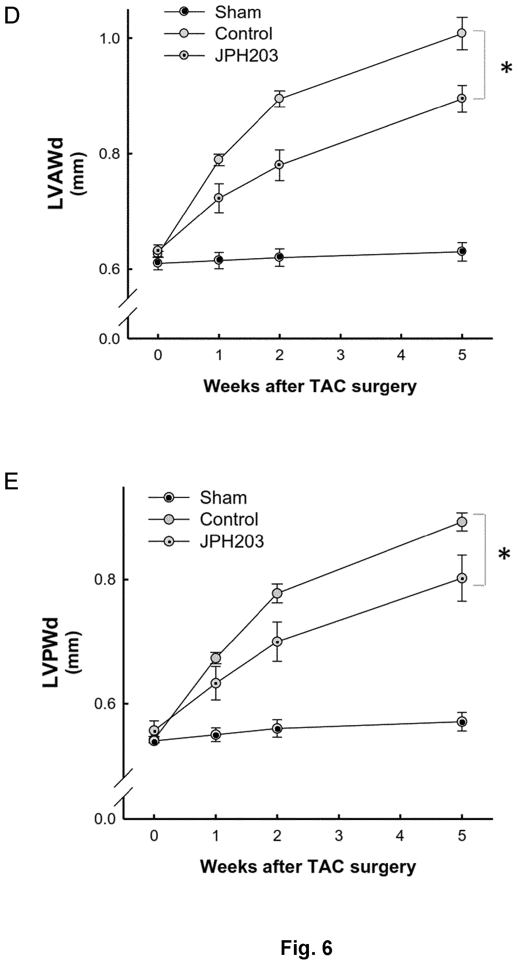

Fig. 6 shows data corresponding to different parameters used to quantify hypertrophy. Fig. 6A: ratio heart weight/tibia length (HW/TL) expressed in mg/mm measured after 5 weeks of aortic stenosis in the different groups described in Fig. 1. Fig. 6B: expression of myosin heavy chain beta (Myh7) and alpha (Myh6) isoforms and expressed as a ratio. Fig. 6C: expression of brain natriuretic peptide (BNP). Expression of these genes was measured by RT-PCR. Figs. 6D and 6E show the kinetics of left ventricular anterior wall (LVAW) and left ventricular posterior wall (LVPW) respectively. Both parameters were measured in diastole by echocardiography at baseline and after 1, 2 and 5 weeks of TAC surgery. Data are presented as mean±SEM. Groups were compared by using one-way analysis of variance. (ANOVA) followed by Tukey HSD test. *p<0.05 respect to Sham group; ^{$}p<0.05 respect to control group.

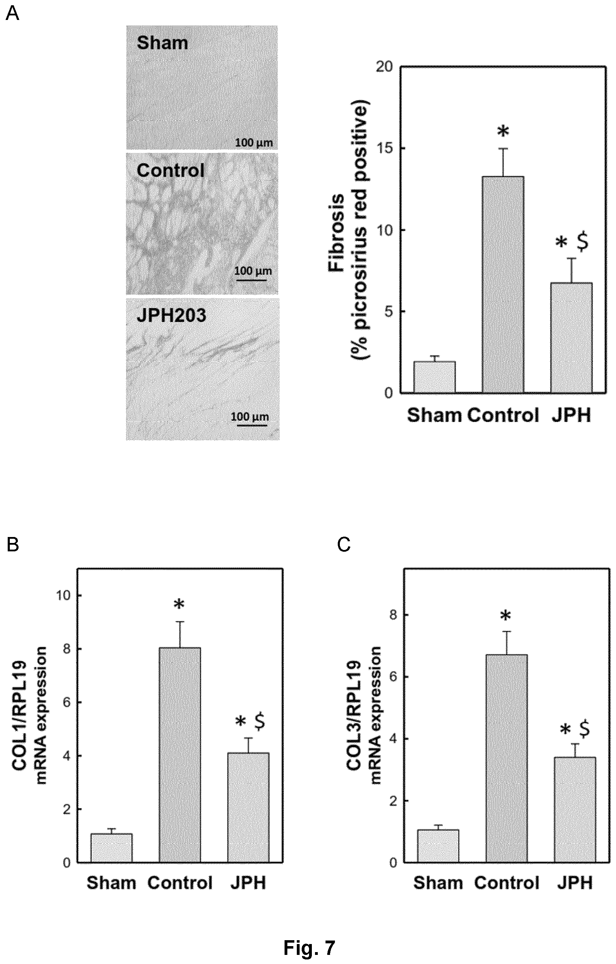

Fig. 7 presents the quantification of fibrosis induced by aortic stenosis (TAC surgery) in mice treated and not treated with JPH203. Fig. 7A shows representative images from transversely sectioned heart slices showing the interstitial deposition of collagen stained with Picrosirius Red and the quantification of the area positive for Picrosirius Red. Fig. 7B and Fig. 7C show the expression of collagen 1 (COL1) and collagen 3 (COL3) respectively, measured by RT-PCR and normalised against the housekeeping gen RPL19. Data are presented as mean±SEM. Groups were compared by using one-way analysis of variance. (ANOVA) followed by Tukey HSD test. *p<0.05 respect to Sham group; ^{$}p<0.05 respect to control group.

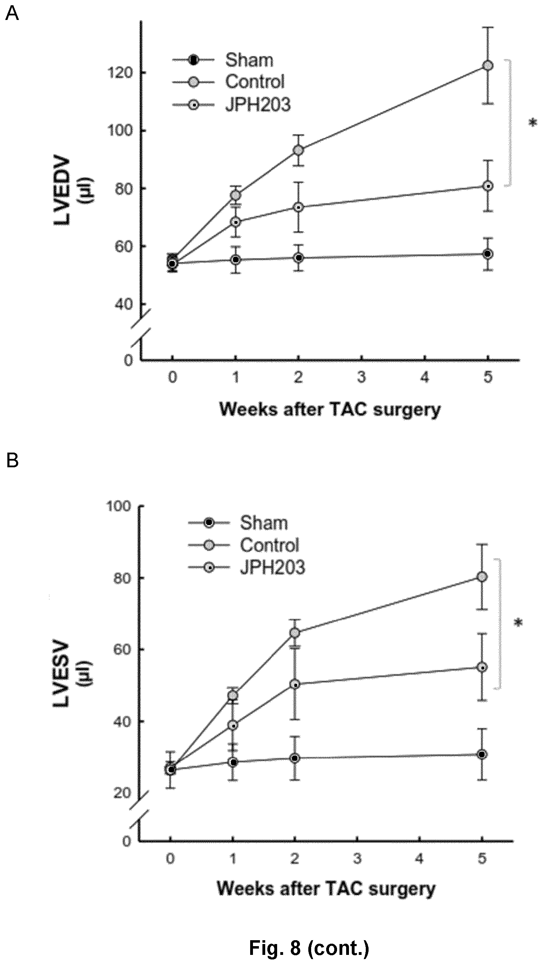

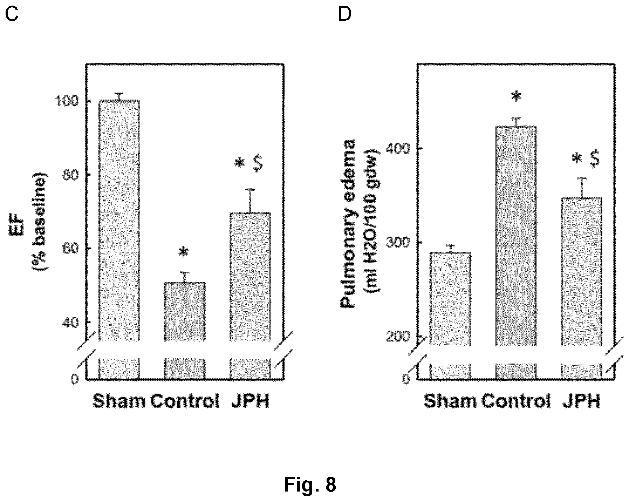

Fig. 8 illustrate the evaluation of heart remodelling and cardiac function by echocardiography and pulmonary oedema in the different groups described in Fig. 1. Figs. 8A and 8B correspond to the kinetics of left ventricular end-diastolic (LVEDV) and end-systolic (LVEDV) volumes respectively, measured by echocardiography at baseline and after 1, 2 and 5 weeks of TAC surgery. Fig. 8C shows the quantification of ejection fraction (EF) measured after 5 weeks of TAC surgery and expressed as percentage with respect basal values. Fig. 8D show the lung oedema expressed as the water content (in ml) in 100 g of dry lung tissue. Data are presented as mean±SEM. Groups were compared by using one-way analysis of variance. (ANOVA) followed by Tukey HSD test. *p<0.05 respect to Sham group; ^{$}p<0.05 respect to control group.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Thus, as used herein, the singular forms "a", "an", and also the definite article "the" include plural referents unless the context clearly dictates otherwise.

As used herein, the term "stereoisomer" refers to all isomers of individual compounds that differ only in the orientation of their atoms in space. The term stereoisomer includes mirror image isomers (enantiomers), mixtures of mirror image isomers (racemates, racemic mixtures), geometric isomers (cis/trans or syn/anti), and isomers that are not mirror images of one another (diastereoisomers). The present invention relates to each of these stereoisomers and also to mixtures thereof.

JPH203 has one chiral centre that can give rise to two stereoisomers, particularly, to two enantiomers. In particular, for the compound JPH203 the term "stereoisomer" includes mirror image isomers (enantiomers) and mixtures of mirror image isomers (racemates, racemic mixtures).

In general, diastereoisomers and enantiomers can be separated by conventional techniques such as chromatography or fractional crystallization. In particular, enantiomers can be individually obtained using enantiospecific synthesis or can be resolved by conventional techniques of optical resolution to give optically pure isomers.

As used herein, the term "sham" refers to the members of a control group that are used to mimic a procedure or treatment without the actual use of the procedure or test substance.

For "sustained myocardial stress" it is to be understood that myocardium is in a condition impairing its normal function due to a particular cause, in particular a pathology not necessarily stemming in the myocardium itself, but also due to abnormalities in coronary arteria and other vascular diseases. In any case, it is a damaging stress that derives from the persisting of some initially compensatory conditions for continuing ejecting blood. An example is the stress leading to adverse ventricular remodelling, wherein initially some parameters (at molecular and cell level) of the myocytes are altered for compensating a loss of function, such as the loss of other myocytes due to ischemia, said parameters evolving to a different pathological condition; the adverse ventricular remodelling.

The expression "prolonged or sustained use of a LAT1 selective inhibitor" means that said inhibitor is chronically administered, thus not only once. In the present disclosure this expression is used as a synonymous of chronic administration. Thus, it is used and usable for a more than one day administration, in particular according to a schedule prescribed by a facultative, which can be a daily schedule, and in particular for the rest of the subject life.

The expression "LAT1 selective inhibitor" is to be understood as any substance capable to selectively reduce LAT1 activity over other members of the system L family (i.e. over LAT2, LAT3 and LAT4) and that is given to a subject, as an external element, in any pharmaceutical form.

The expression "therapeutically effective amount" as used herein, refers to the amount of a compound that, when administered, is sufficient to prevent development of, or alleviate to some extent, one or more of the symptoms of the disease which is addressed. The particular dose of compound administered according to this disclosure will of course be determined by the particular circumstances surrounding the case, including the compound administered, the route of administration, the particular condition being treated, and the similar considerations.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity, or other problems or complications commensurate with a reasonable benefit/risk ratio.

The term "pharmaceutically acceptable salt" used herein encompasses any salt formed from pharmaceutically acceptable non-toxic acids including inorganic or organic acids. There is no limitation regarding the salts, except that if used for therapeutic purposes, they must be pharmaceutically acceptable.

The preparation of pharmaceutically acceptable salts of a compound such as a LAT1 selective inhibitor susceptible to form a salt (i.e., containing a basic or acidic moiety) can be carried out by methods known in the art. For instance, they can be prepared from the parent compound by conventional chemical methods. Generally, such salts are, for example, prepared by reacting the free acid or base forms of these compounds with a stoichiometric amount of the appropriate pharmaceutically acceptable base or acid in water or in an organic solvent or in a mixture of them. A LAT1 selective inhibitor may differ from its salts in some physical properties, but they are equivalent for the purposes of the present disclosure.

Non-limiting examples of pharmaceutically acceptable salts of the LAT1 selective inhibitors which contain a basic centre such as JPH203 include acid addition salts such as the hydrochloride, but also any other pharmaceutically acceptable salts of other acids such as hydrobromic, hydrofluoric, sulphuric, phosphoric, acetic, citric, fumaric, gluconic, lactic, maleic, succinic or tartaric acid. In a preferred embodiment, the pharmaceutically acceptable salt is the hydrochloride salt. The LAT1 selective inhibitors can also provide pharmaceutically acceptable metal salts, in particular non-toxic alkali metal salts, with bases. Examples include the sodium and potassium salts.

LAT1 selective inhibitors or their stereoisomers and/or salts may be in crystalline form either as free solvation compounds or as solvates (e.g. hydrates) and it is intended that both forms are within the scope of the present disclosure. Methods of solvation are generally known within the art. In general, the solvated forms with pharmaceutically acceptable solvents such as water, ethanol and the like are equivalent to the unsolvated form for the purposes of the present disclosure.

In all embodiments of the present disclosure referring to a LAT1 selective inhibitor such as JPH203, its pharmaceutically acceptable salts, or any stereoisomer or mixtures of stereoisomers, either of the compound or of its pharmaceutically acceptable salts, are always contemplated even if they are not specifically mentioned.

As mentioned above, a first aspect of the present disclosure relates to a LAT1 selective inhibitor for use in the prophylactic or the therapeutic treatment either of adverse ventricular remodelling (AVR) or of heart failure.

AVR includes adverse ventricular hypertrophy, adverse ventricular fibrosis, or both of them. Therefore, in an embodiment, AVR is adverse ventricular hypertrophy. In another embodiment, AVR is adverse ventricular fibrosis. In another embodiment, AVR is adverse ventricular hypertrophy and adverse ventricular fibrosis.

In an embodiment, AVR is caused by sustained myocardial stress in a cardiopathy. In another particular embodiment, the cardiopathy is selected from the group consisting of a coronary artery disease (CAD), also known as ischemic heart disease, hypertensive heart disease, rheumatic heart disease, cardiomyopathy (in particular dilated cardiomyopathy), heart arrhythmia, congenital heart disease, cardiac valve dysfunction, and combinations thereof.

More in particular, AVR is caused by a CAD selected from the group consisting of myocardial infarction, left ventricular aneurysm and ischemic cardiomyopathy.

In yet another particular embodiment, the LAT1 inhibitor is for use in the treatment of adverse post-infarction ventricular remodelling (post-MI-AVR). That is, CAD causing AVR is the sustained myocardial stress accompanying a myocardial infarct.

In another particular embodiment of the first aspect of the present disclosure, the LAT1 selective inhibitor is for a sustained or prolonged use. Thus, it is used and usable for a more than one day administration, in particular according to a schedule prescribed by a facultative, which can be a daily schedule, and in particular for the rest of the subject life.

In another particular embodiment, optionally in combination with any of the embodiments above or below, the LAT1 inhibitor is for a sustained or prolonged use and for AVR induced by hypertension. When AVR is caused by hypertension, a particular administration schedule comprises: administering daily the calpain inhibitor at appropriate dose at the moment when the cardiologist detects the first symptoms of ventricular remodelling,

In another embodiment, optionally in combination with one or more features of the various embodiments described above or below throughout all the description, the LAT1 selective inhibitor is selected from the group consisting of:
JPH203 (nanvuranlat), triiodothyronine,
(S)-2-amino-3-(3-((2,4-dicyano-3-(4-(2-(methylamino)-2-oxoethoxy)phenyl)benzo[4,5]imidazo[1,2-a]pyridin-1-yl)carbamoyl)phenyl)propanoic acid 1 (KMH-233),
(S)-2-am ino-3-(3, 5-dichloro-4-((7-phenylnaphthalen-1-yl)methoxy)phenyl)propanoic acid (SKN101),
(S)-2-amino-3-(4-((7-(3-aminophenyl)naphthalen1-yl)methoxy)-3,5-dichlorophenyl)propanoic acid (SKN103),
(S)-2amino-3-(4-((7-(4-aminophenyl)naphthalen-1-yl)methoxy)-3,5dichlorophenyl)propanoic acid (SKN104),
(S)-2-amino-3-(3,5dichloro-4-((7-(3-sulfophenyl)naphthalen-1-yl)methoxy)phenyl) propanoic acid (SKN105),
(Z)-4-chloro-N-(4(trifluoromethoxy)phenyl)-5H-1,2,3-dithiazol-5-imine;
(S)-2-(5-(Benzyloxy)-1H-indol-3-yl)-1-carboxyethan-1-amine · HCl; 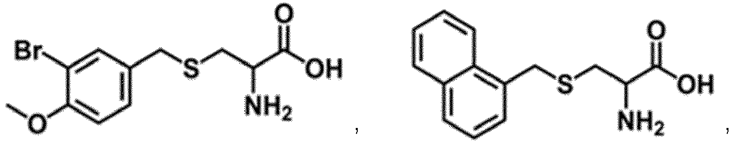 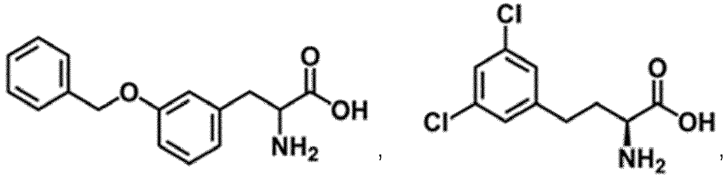 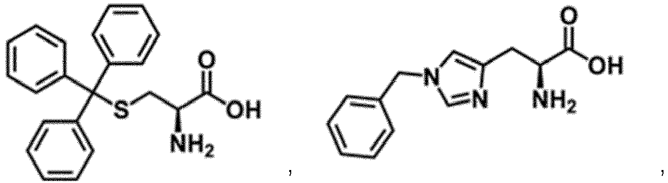 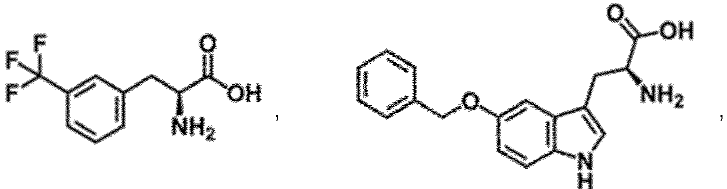 and

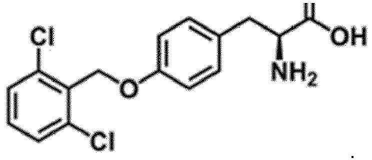

All the abovementioned LAT1 selective inhibitors are commercially available (see Kongpracha, P. et al. "Structure-activity relationship of a novel series of inhibitors for cancer type transporter L-type amino acid transporter 1 (LAT1)". J Pharmacol Sci. 2017; vol. 133(2), pp. 96-102; Singh N. et al. "Discovery of Potent Inhibitors for the Large Neutral Amino Acid Transporter 1 (LAT1) by Structure-Based Methods". Int J Mol Sci. 2018; vol. 20(1), pp. 1-27). The preparation of (S)-2-(5-(Benzyloxy)-1H-indol-3-yl)-1-carboxyethan-1-amine . HCl is disclosed in Graff, J. et al. "The Evaluation of I-Tryptophan Derivatives as Inhibitors of the I-Type Amino Acid Transporter LAT1 (SLC7A5)". ChemMedChem. 2022, 17.

In a particular embodiment, the LAT1 selective inhibitor for use in the prophylactic or the therapeutic treatment of adverse ventricular remodelling or in the prophylactic or the therapeutic treatment of heart failure is JPH203, particularly JPH203 in the form of its dihydrochloride salt.

Other potential LAT1 selective inhibitors can be found by any one of the methods disclosed in Kongpracha, P. *et al.* (*ibid.*) or Singh N. *et al.* (*ibid*.). As an instance, the potential inhibitory effects of a compound can be examined on the LAT1-mediated uptake of L-[¹⁴C]leucine (1 mM) in HEK293-hLAT1 cells and the LAT2-mediated uptake of L-[¹⁴C]alanine (1 mM) in HEK293-hLAT2 cells as disclosed in Kongpracha, P. *et al.* (*ibid*.), and by comparing the inhibitory effect with those of 2-amino-2-norbornanecarboxylic acid (BCH), a conventional system L inhibitor, and T3, a LAT1 selective inhibitor.

As mentioned above, a third aspect of the present disclosure relates to a drug combination comprising a LAT1 selective inhibitor as defined above and a therapeutic agent selected from the group consisting of an angiotensin-converting enzyme (ACE) inhibitor, an angiotensin receptor neprilysin inhibitor (ARNi), a beta-adrenergic receptor inhibitor, a mineralocorticoid receptor antagonist, and a sodium-glucose cotransporter type 2 (SGLT2) inhibitor as defined herein.

In one embodiment, optionally in combination with one or more features of the various embodiments described above, the drug combination comprises a LAT1 selective inhibitor as defined above and an ACE inhibitor.

Non-limiting examples of ACE inhibitors include enalapril, captopril, benazepril, fosinopril, lisinopril, moexipril, perindopril, quinapril, ramipril, trandolapril.

In one embodiment, optionally in combination with one or more features of the various embodiments described above, the drug combination comprises a LAT1 selective inhibitor as defined above and an angiotensin receptor neprilysin inhibitors.

Non-limiting examples of angiotensin receptor neprilysin inhibitors include sacubitril, sacubitril/valsartan, sacubitrilat, omapatrilat, ecadotril, and candoxatril.

In one embodiment, optionally in combination with one or more features of the various embodiments described above, the drug combination comprises a LAT1 selective inhibitor as defined above and a beta-adrenergic receptor inhibitors.

Non-limiting examples of beta-adrenergic receptor inhibitors include bisoprolol, carvedilol, metoprolol, propranolol, nadolol, pindolol, labetalol, penbutolol, sotalol, timolol, atenolol, acebutolol, betaxolol, esmolol, and nebivolol.

In one embodiment, optionally in combination with one or more features of the various embodiments described above, the drug combination comprises a LAT1 selective inhibitor as defined above and a mineralocorticoid receptor antagonists.

Non-limiting examples of mineralocorticoid receptor antagonists include spironolactone, and eplerenone.

In one embodiment, optionally in combination with one or more features of the various embodiments described above, the drug combination comprises a LAT1 selective inhibitor as defined above and a sodium-glucose cotransporter type 2 inhibitors.

Non-limiting examples of sodium-glucose cotransporter type 2 inhibitors include empaglifozin dapagliflozin, ertugliflozin, and canagliflozin.

### Pharmaceutical compositions and kits of parts

The present disclosure also relates to pharmaceutical compositions comprising a LAT1 selective inhibitor as defined above, as well as to pharmaceutical compositions and kits of parts comprising the drug combinations defined herein.

Accordingly, the disclosure relates to a pharmaceutical composition comprising a therapeutically effective amount of a LAT1 selective inhibitor as defined above, together with one or more pharmaceutically acceptable excipients or carriers, for use in the prophylactic or the therapeutic treatment of adverse ventricular remodelling and/or of heart failure.

In a particular embodiment, optionally in combination with any of the embodiments above, the pharmaceutical composition as defined herein is adapted for oral use.

Thus, the pharmaceutical composition can be formulated for oral administration and can contain one or more physiologically compatible carriers or excipients, in solid or liquid form. For example, formulations suitable for oral administration may include liquid solutions, suspensions, capsules, sachets or tablets, emulsions or dry powdered forms suitable for reconstitution with water.

In another particular embodiment, optionally in combination with any of the embodiments above, the pharmaceutical composition as defined herein is adapted for intravenous use.

The pharmaceutical composition may also be formulated for intravenous administration in combination with conventional injectable liquid carriers, such as water or suitable alcohols. Conventional pharmaceutical excipients for injection, such as stabilizing agents, solubilizing agents, and buffers, may be included in such compositions.

As an example, intravenous compositions can comprise the LAT1 selective inhibitor as defined above and 2-hydroxypropyl-beta-cyclodextrin in a solvent, particularly water, wherein the percentage by weight of 2-hydroxypropyl-beta-cyclodextrin in relation to said solvent is from 20 % (w/w) to 60 % (w/w). More in particular the percentage by weight of 2-hydroxypropyl-beta-cyclodextrin in relation to said solvent is from 30 % (w/w) to 50% (w/w). More in particular, 40 % (w/w). In another particular embodiment, optionally in combination with any of the embodiments above or below, the LAT1 selective inhibitor in the intravenous compositions is in an amount for a dosage from 5 mg/kg/day to 50 mg/kg/day, more in particular from 8 mg/kg to 25 mg/kg, and more in particular 12.5 mg/kg/day, all intervals including the extremes.

In one embodiment, optionally in combination with one or more features of the various embodiments described above or below throughout all the description, the LAT1 selective inhibitor as defined above is the only active ingredient of the composition.

The disclosure also relates to a single pharmaceutical composition which comprises: a) a therapeutically effective amount of a LAT1 selective inhibitor as defined above; b) a therapeutically effective amount of a therapeutic agent selected from the group consisting of a angiotensin-converting enzyme (ACE) inhibitor, an angiotensin receptor neprilysin inhibitor (ARNi), a beta-adrenergic receptor inhibitor, a mineralocorticoid receptor antagonist, and a sodium-glucose cotransporter type 2 (SGLT2) inhibitor; and one or more pharmaceutically acceptable excipients or carriers.

As used herein, the term "single pharmaceutical composition" refers to a dosage form that contains both a) and b) in the same composition.

The present disclosure also relates to a kit of parts comprising: a) a first pharmaceutical composition which comprises a therapeutically effective amount of a LAT1 selective inhibitor as defined above, together with one or more pharmaceutically acceptable excipients or carriers; and a) a second pharmaceutical composition which comprises a therapeutically effective amount of a therapeutic agent as defined herein above, together with one or more pharmaceutically acceptable excipients or carriers; wherein compositions i) and ii) are separate compositions.

For the purposes of the present disclosure, the term "kit-of-parts or package" refers to a combined preparation, wherein pharmaceutical compositions a) and b) are physically separated and are packaged or marked for use together. In the context of the present disclosure "for use together or in combination" does not limit the order in which the pharmaceutical compositions are administered. Thus, pharmaceutical composition comprising a LAT1 selective inhibitor as defined above may be administered prior to, concomitantly with, or subsequent to the administration of the pharmaceutical composition comprising one or more therapeutic agents as defined above.

Suitable pharmaceutical compositions to be used in the present disclosure are well-known in the art. The election of the pharmaceutical formulation will be determined by the skilled person depending upon the nature of the active compounds present in the composition, and its route of administration. In particular, oral or intravenous routes of administration can be used.

For being soluble in water, the LAT1 selective inhibitor as defined above is preferably administered in the form of a water soluble pharmaceutically acceptable salt, such as in the form of its dihydrochloride.

### Uses

The LAT1 selective inhibitor as defined above, as well as the drug combinations disclosed herein, are useful in the treatment of adverse ventricular remodelling and of heart failure.

Thus, as mentioned above, a first aspect of the present disclosure relates to a LAT1 selective inhibitor as defined above as defined above for use in the prophylactic or the therapeutic treatment of adverse ventricular remodelling and of heart failure.

This aspect may also be formulated as the use of a LAT1 selective inhibitor as defined above such as JPH203 or a pharmaceutically acceptable salt thereof, or any stereoisomer or mixtures of stereoisomers of any of them as defined above for the manufacture of a medicament for the prophylactic or the therapeutic treatment of adverse ventricular remodelling or of heart failure. It also forms part of the invention a method for the prophylactic or the therapeutic treatment either of adverse ventricular remodelling or of heart failure, comprising administering to a subject in need thereof, including a human, a pharmaceutically effective amount of a LAT1 selective inhibitor as defined above such as JPH203, or any stereoisomer or mixtures of stereoisomers of any of them, together with one or more pharmaceutically acceptable excipients or carriers.

In all embodiments of the present disclosure, when referring to being for use in the prophylactic or the therapeutic treatment either of adverse ventricular remodelling (AVR) or of heart failure, should be understood to include all the particular embodiments relates to the specific diseases even if they are not specifically mentioned. Hence, for use in the prophylactic or the therapeutic treatment of AVR include the particular embodiments wherein AVR is caused by sustained myocardial stress in a cardiopathy; wherein AVR is caused by sustained myocardial stress in a cardiopathy, particularly, wherein the cardiopathy is selected from the group consisting of a coronary artery disease (CAD), also known as ischemic heart disease, hypertensive heart disease, rheumatic heart disease, cardiomyopathy (in particular dilated cardiomyopathy), heart arrhythmia, congenital heart disease, cardiac valve dysfunction, and combinations thereof.

More in particular, AVR is caused by a CAD selected from the group consisting of myocardial infarction, left ventricular aneurysm and ischemic cardiomyopathy.

In yet another particular embodiment, the LAT1 inhibitor is for use in the treatment of adverse post-infarction ventricular remodelling (post-MI-AVR). That is, CAD causing AVR is the sustained myocardial stress accompanying a myocardial infarct.

Throughout the description and claims the word "comprise" and variations of the word, are not intended to exclude other technical features, additives, components, or steps. Furthermore, the word "comprise" encompasses the case of "consisting of".

The following examples are provided by way of illustration, and they are not intended to be limiting of the present disclosure. Furthermore, the present disclosure covers all possible combinations of particular and preferred embodiments described herein.

### EXAMPLES

### METHODS

### Transverse Aortic Constriction (TAC) mouse model

The transverse aortic constriction (TAC) model increases the left ventricle afterload, which clinically is produced by aortic stenosis or systemic hypertension.

Twelve weeks C57BL/6N old mice were anesthetised with ketamine/xylazine (100 mg/kg and 10 mg/kg, respectively) and mechanically ventilated (SAR-830/AP, CWE). The transverse aortic arch was ligated by tying a 6-0 silk suture around the aorta and a 27G needle to yield a partial and reproducible constriction when the needle was removed. In sham animals, the aortic arch was visualized but not banded. Thorax was closed and analgesia was administrated (buprenorphine at 0.1 mg/kg every 12 hours until 48 hours). Mice were euthanized by lethal intraperitoneal dose of sodium pentobarbital (100 mg/kg) (Cat. No. 4579, Tocris).

### JPH203 administration

The LAT1 inhibitor JPH203 (Cat. No. S8667) was intraperitoneally (ip) and chronically administered, starting the following day after TAC surgery.

Mice were randomly distributed to receive JPH203 at 12.5 mg/kg/day (prepared at 2 mg/ml of 40% 2-hydroxipropil-beta-cyclodextrin in saline). Sham and control groups received JPH203 vehicle (40% 2-hydroxipropil-beta-cyclodextrin in saline). Mice were euthanized 2 or 5 weeks after surgery.

The JPH203 concentration selected was based on manufacturer's recommendations.

### Echocardiographic parameters

Mice underwent transthoracic echocardiography at baseline (before surgery) and at 1, 2 and 5 weeks after surgery. Echocardiography was performed using a Prospect T1 Preclinical High Frequency Ultrasound System with a PB406e Probe 40MHz transducer (S-Sharp) applied to the shaved chest wall of mice anesthetized with isoflurane. The ejection fraction (EF), the left ventricular end-systolic (LVESd) and diastolic (LVEDd) internal diameters, left ventricular anterior wall (LVAW) thickness and left ventricular posterior wall (LVPW) thickness were measured in M-mode recordings.

### Hypertrophy

Hypertrophy was calculated as heart weight-to-tibia length ratio. Echocardiographic parameters like IVS and LVPW thicknesses, as well as BNP and Myh7 mRNA levels, are the common parameters used to define myocardial hypertrophy.

### Fibrosis

Hearts were fixed in buffered 4% paraformaldehyde and embedded in paraffin for histological evaluation. The amount of interstitial collagen deposition was determined in 4 µm transverse sections at the papillary muscles level stained with Picrosirius red (Sigma-Aldrich). At least ten random photomicrographs per animal were taken with the Leica DM IRBE Inverted Fluorescence Microscope at x200 magnification. The analysis was performed using Image J 1.50 Software. Interstitial fibrosis was expressed as the percentage of fibrotic area with respect to total area under quantification, perivascular fibrosis was manually excluded from the analysis.

### Pr-qPCR

Total RNA was extracted from heart samples using the NZY Total RNA Isolation kit (Cat. No. MB13402, NZY Tech) following the manufacturer's protocol.

RNA was reverse transcribed into cDNA using a High-Capacity cDNA Reverse Transcription kit (Cat. No. 4368814, Applied Biosystems) following manufacturer's instructions. The thermal cycler (Bio-Rad) was programmed to run a single cycle of 25 °C for 10 min, 37 °C for 120 min, 85 °C for 5 min and kept at 4 °C until use.

Quantitative RT-PCR was performed using TaqMan^{™} Gene Expression Master Mix (Cat. No. 4369016, Applied Biosystems). The amplification program consisted of 50 °C for 2 min, 95 °C for 10 min and 40 cycles of 95 °C for 15 s and 60 °C for 1 min and was performed in a LightCycler^{®}480 Real-Time PCR System (Roche Diagnostics). Primers used for BNP, Myh7, Col1A1, Col3A1, SLC7A5, SLC3A2, BCKDHB, PP1MK, and RPL19 were purchased from ThermoFisher (Mm01255770_g1, Mm01249941_m1, Mm00600555_m1, Mm01254476_m1, Mm00441516_m1, Mm00500521_m1, Mm01177077_m1, Mm00615792_m1 and Mm02601633_g1, respectively).

### Western Blot

Pulverized frozen hearts were homogenized in RIPA buffer containing 50 mM Tris-HCl, 150 mM NaCl, 10 mM EDTA, 0.1 % SDS, 0.5% Na-deoxycholate, 1% Triton X-100, 1 mM DTT, 10 mM NaF, 2 mM Na₃VO₄ and 1% Protease Inhibitor Cocktail (Sigma) (pH 7.4).

Proteins were separated by electrophoresis on a 10% SDS gel, transferred onto 0.45 µm pore nitrocellulose membrane (Amersham^{™} Protran^{®}), and immunoblotted with primary antibodies raised against Slc7a5 (ANT-105, Alomone) and SDHA (ab14715, Abcam). Protein bands were detected by chemiluminiscence (SuperSignal^{™} West Femto, Cat. No. 34095) using a charge-coupled device system (Image Reader LAS-4000, Fujifilm) and analysed with the Image Studio Lite 5.2 Software. Equal protein load was confirmed by Ponceau staining.

### Ventricular cardiomyocytes and fibroblasts isolation by Langendorff system

Mouse ventricular fibroblasts (FB) and calcium tolerant rod-shaped cardiomyocytes were isolated on a Langendorff system. The heart was washed with calcium-free modified Krebs solution (100 mM NaCl, 10 mM HEPES sodium salt, 2.6 mM KCl, 1.2 mM MgSO₄, 1.2 mM KH₂PO₄, 11mM glucose, 10 mM 2,3-Butanedione monoxime pH 7.4) at 37 °C. Extracellular matrix was digested for 15 minutes by adding 0.03% of type 2 collagenase (Worthington, Biochemical Corporation) and 15 µM CaCl₂ to the krebs solution. Then, atria were removed and the heart was minced into small pieces in the same enzymatic solution for 10 minutes by pipetting up and down with a serological pipette. Undigested tissue and debris were removed by filtering the prior cell suspension through a 180 µm pore size mesh, and centrifuging at 25 g for 3 minutes. The pellet rich in cardiomyocytes was subjected to gradual normalization of calcium concentration up to 1 mM CaCl₂ in order to select rod-shaped calcium-tolerant cardiomyocytes. The supernatant was meshed through a 40 µm cell stainer (ThermoFisher) and centrifuged at 650 g for 5 minutes. The pellet, which contained the FB fraction, was stored at -80 °C.

### Isolated cardiomyocytes immunofluorescence

Calcium-tolerant cardiomyocytes are resuspended in M199 medium supplemented with 4% foetal bovine serum (FBS) and seeded in pre-coated 8-well culture slide (Cat. No. 354118, Corning) with 5% laminin. Medium is changed after 1 hour of incubation to discard non-attached cells.

Cardiomyocytes were fixed in 4% PFA for 15 min at room temperature (RT), permeabilized with 0.1% Triton X-100 in blocking buffer (1% BSA in PBS) for 15 min at RT and incubated with blocking buffer for 1 hour at RT. Primary antibodies raised against Slc7a5 (ANT-105, Alomone) at 1:200 in blocking buffer, and Slc7a5 together with its blocking peptide (BLP-NT105, Alomone) at 1:4 ratio were left O/N at 4 °C. The next day, the cells were incubated with secondary antibodies goat anti-rabbit conjugated to Alexa Fluor^{™} 594 (Cat. No. A-11012) at 1:500 in blocking buffer for 1 hour at RT. Finally, nuclei were stained with Hoescht 33342 at 5 µg/mL for 5 min and slides were mounted with Prolong^{™} Gold antifade mounting reagent (Cat. No. P10144, Invitrogen).

Z-stack images were acquired with the spectral FluoView-1000 Olympus confocal microscope at x400 magnification.

### Microarray-based gene expression profile

For determination of differential gene expression, RNA was isolated, as previously described, from mice myocardial samples and its quality was analysed by capillary electrophoresis (Bioanalyzer 2100, Agilent) and amplified using the WT Plus Reagent Kit-HT (Applied Biosystems, Thermo Fisher Scientific). Microarray service was done by the Molecular Diagnosis and Genomic platforms of the High Technology Unit (VHIR) using Clariom S type arrays specific for mouse transcriptome (plate format) (Applied Biosystems). The cartridge arrays were scanned using the GeneChip Scanner 3000 system (Affymetrix) and a GeneTitan MultiChannel equipment was used for array plates. Data was transferred to the Statistics and Bioinformatics Unit (VHIR) for its analysis which included three main steps.
1) Quality control and pre-processing of the arrays used.
2) Selection of differentially expressed genes.
3) Analysis of biological significance. These analyses were carried out with the statistical program R (Copyright 2019 The R Foundation for Statistical Computing) and the specific packages developed for the analysis of microarrays found in the Bioconductor repository (www.bioconductor.org).

### LC-MS/MS

After 5 hours of fasting, mice were injected with an intraperitoneal dose of L-Leucine-¹³C₆, ¹⁵N (Cat. No. 608068, Sigma-Aldrich) at 0.1 mg/g 30 minutes before euthanizing with a lethal injection of sodium pentobarbital resuspended in saline and injected at 100 mg/kg (Cat. No. 4579, Tocris).

The levels of Branched-Chain Amino Acids (BCAA) and their main metabolites in biological samples will be determined by Liquid Chromatography tandem Mass Spectrometry (LC-MS/MS). Briefly, the determination of amino acids (BCAA but also amino acids involved in glutaminolysis) was performed by using a hydrophilic interaction chromatography (HILIC) with an amide column. After addition of the adequate internal standard (containing labelled analogues), protein precipitation with organic solvents and centrifugation, the supernatant was injected into the LC-MS/MS system operating in a Selected Reaction Monitoring (SRM) method by acquiring two transitions for each compound. The determination of α-ketoacids, α-hydroxy acids and SCFA (including the main BCAA metabolites and other compounds related with glutaminolysis) was performed by oBHA derivatization. The internal standard (containing labelled analogues) was added into the sample and the mixture was derivatized by adding a derivatizing mixture consisting in oBHA:EDC (1:1) in pyridine. After reaction for 1 h at room temperature, 1 mL of milliQ water and 4 mL of ethyl acetate was added to perform a liquid-liquid extraction. The organic layer was separated and dried under a nitrogen stream, the extracts reconstituted in mobile phase and injected into the LC-MS/MS system operating in a Selected Reaction Monitoring (SRM) method by acquiring two transitions for each compound. Quantification was performed by means of external calibration using authentic referent material. The ratio area of analyte/area of internal standard was used as response for determination of the concentration levels. Several quality control samples (QCs) were analysed with every analytical batch with the aim of checking the inter-batch robustness of the results. Samples were reanalysed if QC results are systematically above acceptable criteria (80-120%).

### RESULTS

### Myocardial remodelling induced by aortic stenosis increases LAT1 expression and inhibits branched-chain amino acid (BCAA) catabolism

Data obtained from a microarray analysis performed using mRNA extracted from mouse hearts subjected to TAC surgery for 5 weeks and compared to sham mice, demonstrate that chronic stress caused by aortic stenosis (TAC surgery) induces a significant increase in the expression of Slc7a5 (LAT1) (Figure 2A). The overexpression of LAT1 was confirmed by confocal analysis in cardiomyocytes (Figure 2B) and by RT-PCR in fibroblasts (Figure 2C) isolated from mouse hearts after 5 weeks of TAC surgery.

In addition to changes in the expression of LAT1, which mediates the uptake of amino acids into the heart, the data obtained from the same microarray also demonstrates that aortic stenosis produces an overall downregulation of genes that express mitochondrial enzymes involved in the catabolism of amino acids (Figure 3A). The scheme shown in Figure 3B indicate that these downregulated enzymes are critical steps in the conversion of BCAA into compounds used by the Krebs cycle.

### Administration of JPH203 attenuates amino acid uptake and reverses the inhibition of branched chain amino acid catabolism induced by aortic stenosis

Microarray analysis comparing the expression of genes involved in BCAA catabolism between sham and JPH203 treated mice, demonstrate that JPH203 administration attenuates the downregulation of genes involved in BCAA catabolism induced by TAC surgery (Figure 4A). The microarray results were confirmed by RT-PCR quantification of the expression of BCKDHB (Figure 4B) and PP1MK (Figure 4C). The reduction in the expression of these two enzymes that play a critical role in the catabolic BCAA pathway observed in control hearts, was significantly attenuated by JPH203 treatment (P=0.015 for BCKDHB and P=0.013 for PP1MK, n=6 per group).

To determine whether the changes in the expression of LAT1 and genes involved in BCAA catabolism measured by microarray and RT-PCR produce alterations in the myocardial BCAA content, BCAA and metabolites derived from BCAA catabolism were measured by LC-MS/MS in heart extracts obtained from the different experimental groups after 2 weeks of TAC surgery (Figure 5). The results demonstrate that aortic stenosis produce a significant increase in the myocardial content of BCAA, as reflected by the enhanced concentration of valine, leucine and isoleucine, that was significantly attenuated by the chronic administration of JPH203 (Figure 5A). To analyse if these global changes in BCAA content are due to changes in BCAA uptake or catabolism, and how JPH203 modulate these processes, mice received 13C,15N-leucine intraperitoneally 30 minutes before heart extraction. Analysis of heart extracts by LC-MC/MC showed a marked increase in the ratio between the plasma and myocardial content of 13C6,15N-leucine in the control group, and a reduction in the levels of the 13C6,15N-leucine metabolites 13C-isovaleric acid and 13C-HMG (Figure 5B). Importantly, the administration of JPH203 prevented these alterations in BCAA metabolism, demonstrating that the LAT1 overexpression occurring during aortic stenosis is responsible of them.

### Chronic administration of JPH203 attenuates cardiac hypertrophy

As it has been previously described, aortic stenosis produced marked heart hypertrophy, reflected by increased HW/TL ratio (Figure 6A), the mRNA expression of common markers of hypertrophy measured by RT-PCR such as the beta isoform of the myosin heavy chain (MyH7; Figure 6B) and brain natriuretic peptide (BNP; Figure 6C), and the progressive increase in anterior and posterior wall thicknesses measured by echocardiography throughout the 5 weeks of aortic stenosis (Figures 6D-E).

Chronic administration of JPH203 produce a significant attenuation of hypertrophy as shown by the significant reduction in all these parameters with respect to the control group (Figure 6): JPH203 attenuated the increase in HW/TL ratio by 33% (P<0.001), the MyH7/MyH6 ratio by 44% (P<0.001), the BNP expression by 65% (P=0.001), and reduced the posterior and anterior wall thicknesses by 28% (P=0.006) and 30% (P=0.026) respectively.

### Chronic administration of JPH203 attenuates cardiac fibrosis

Aortic stenosis induced severe fibrosis, shown as the accumulation of interstitial collagen in transversely sectioned hearts stained with Pricosirius Red (Figure 7A), and confirmed by measuring the expression of collagen I and collagen III by RT-PCR in myocardial homogenates obtained after two weeks of TAC surgery (Figure 7B and 7C respectively). The chronic administration of JPH203 produced a significant attenuation in the expression of collagen I (56%, P=0.003) and collagen III (58%, P=0.002) that resulted in reduced interstitial collagen deposition (57%, P<0.001) (Figure 7).

### Chronic administration of JPH203 attenuates ventricular dilatation and cardiac dysfunction.

Echocardiographic data obtained before TAC surgery and 1, 2 and 5 weeks after TAC surgery showed a progressive dilatation of the ventricular diastolic and systolic volumes (LVEDV, and LVESV respectively). This increase was already significant after one week of TAC surgery (Figure 8A and 8B).

After 5 weeks of TAC surgery, control mice showed signs of heart failure, as evidenced by the deterioration in cardiac function, measured as a reduction in the ejection fraction (EF) and expressed as percentage with respect basal values (Figure 8C), and the development of pulmonary oedema, measured as the water content in lungs obtained after 5 weeks of TAC surgery (Figure 8D).

Chronic administration of JPH203 produced a significant attenuation of the severity of ventricular dilation (Figure 8A-8B). After 5 weeks of TAC surgery, JPH203 reduced the diastolic volume by 60% (P=0.017) and the systolic volume by 51% (P=0.021) with respect to control group. Treatment with JPH203 also produced a significant attenuation of cardiac dysfunction (38%, P=0.030) (Figure 8C) and the development of pulmonary oedema (56%, P<0.001) (Figure 8D) with respect to control group. Altogether, these data demonstrate that JPH203 is effective in attenuating the development of heart failure induced by aortic stenosis.

### REFERENCES CITED IN THE APPLICATION

1. Kongpracha, P. et al. "Structure-activity relationship of a novel series of inhibitors for cancer type transporter L-type amino acid transporter 1 (LAT1)". J Pharmacol Sci. 2017; vol. 133(2), pp. 96-102;
2. Singh N. et al. "Discovery of Potent Inhibitors for the Large Neutral Amino Acid Transporter 1 (LAT1) by Structure-Based Methods". Int J Mol Sci. 2018; vol. 20(1), pp. 1-27;
3. Graff, J. et al. "The Evaluation of I-Tryptophan Derivatives as Inhibitors of the I-Type Amino Acid Transporter LAT1 (SLC7A5)". ChemMedChem. 2022, 17.

## Claims

1. A LAT1 selective inhibitor or a pharmaceutically acceptable salt thereof, or any stereoisomer or mixtures of stereoisomers of any of them, for use in the prophylactic or the therapeutic treatment either of adverse ventricular remodelling or of heart failure.

2. The LAT1 selective inhibitor or a pharmaceutically acceptable salt thereof, or any stereoisomer or mixtures of stereoisomers of any of them, for use according to claim 1, wherein adverse ventricular remodelling is caused by sustained myocardial stress in a cardiopathy.

3. The LAT1 selective inhibitor or a pharmaceutically acceptable salt thereof, or any stereoisomer or mixtures of stereoisomers of any of them, for use according to claim 2, wherein the cardiopathy is selected from the group consisting of a coronary artery disease (CAD), hypertensive heart disease, rheumatic heart disease, cardiomyopathy, heart arrhythmia, congenital heart disease, cardiac valve dysfunction, and combinations thereof.

4. The LAT1 selective inhibitor or a pharmaceutically acceptable salt thereof, or any stereoisomer or mixtures of stereoisomers of any of them, for use according to claim 3, the cardiopathy is a CAD selected from the group consisting of myocardial infarction, left ventricular aneurysm and ischemic cardiomyopathy.

5. The LAT1 selective inhibitor or a pharmaceutically acceptable salt thereof, or any stereoisomer or mixtures of stereoisomers of any of them, for use according to any one of claims 1 to 4, which is selected from the group consisting of:
JPH203 (nanvuranlat), triiodothyronine,
(S)-2-amino-3-(3-((2,4-dicyano-3-(4-(2-(methylamino)-2-oxoethoxy)phenyl)benzo[4,5]imidazo[1,2-a]pyridin-1-yl)carbamoyl)phenyl)propanoic acid 1 (KMH-233),
(S)-2-amino-3-(3,5-dichloro-4-((7-phenylnaphthalen-1-yl)methoxy)phenyl)propanoic acid (SKN101),
(S)-2-amino-3-(4-((7-(3-aminophenyl)naphthalen1-yl)methoxy)-3,5-dichlorophenyl)propanoic acid (SKN103),
(S)-2amino-3-(4-((7-(4-aminophenyl)naphthalen-1-yl)methoxy)-3,5dichlorophenyl)propanoic acid (SKN104),
(S)-2-amino-3-(3,5dichloro-4-((7-(3-sulfophenyl)naphthalen-1-yl)methoxy)phenyl) propanoic acid (SKN105),
(Z)-4-chloro-N-(4(trifluoromethoxy)phenyl)-5H-1,2,3-dithiazol-5-imine;
(S)-2-(5-(Benzyloxy)-1H-indol-3-yl)-1-carboxyethan-1-amine · HCl; 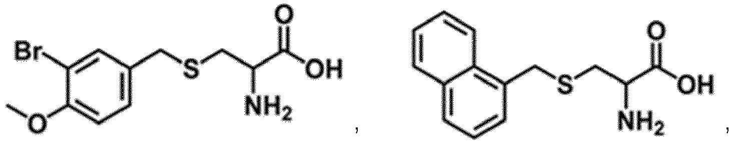 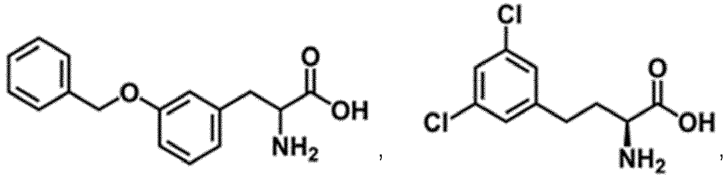 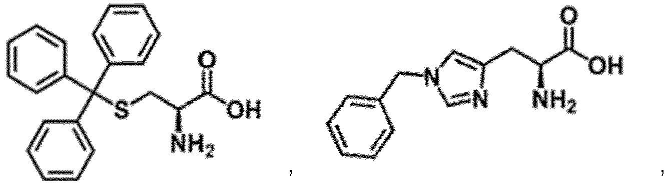 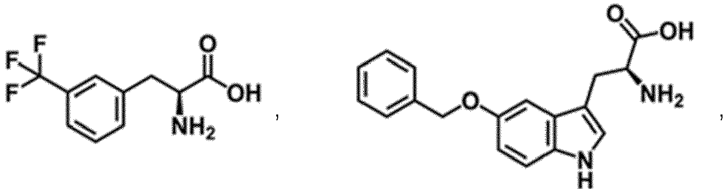 and

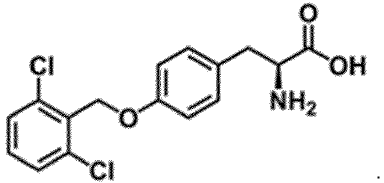

6. The LAT1 selective inhibitor or a pharmaceutically acceptable salt thereof, or any stereoisomer or mixtures of stereoisomers of any of them, for use according to claim 5, which is JPH203, particularly JPH203 in the form of its dihydrochloride salt.

7. A pharmaceutical composition comprising a therapeutically effective amount of a LAT1 selective inhibitor or a pharmaceutically acceptable salt thereof, or any stereoisomer or mixtures of stereoisomers of any of them, as defined in any one of claims 1 to 6, together with one or more pharmaceutically acceptable excipients or carriers, for use in the prophylactic or the therapeutic treatment of adverse ventricular remodelling and/or of heart failure.

8. The pharmaceutical composition according to claim 7, which is adapted either for oral use or for intravenous use.

9. A drug combination comprising:
a) a LAT1 selective inhibitor or a pharmaceutically acceptable salt thereof, or any stereoisomer or mixtures of stereoisomers of any of them, and
b) a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor.

10. A single pharmaceutical composition which comprises:
a) a therapeutically effective amount of JPH203 or a pharmaceutically acceptable salt thereof, or any stereoisomer or mixtures of stereoisomers of any of them;
b) a therapeutically effective amount of a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor;
and one or more pharmaceutically acceptable excipients or carriers.

11. A kit of parts comprising:
ii) a first pharmaceutical composition which comprises a therapeutically effective amount of a LAT1 selective inhibitor or a pharmaceutically acceptable salt thereof, or any stereoisomer or mixtures of stereoisomers of any of them, together with one or more pharmaceutically acceptable excipients or carriers; and
ii) a second pharmaceutical composition which comprises a therapeutically effective amount of a therapeutically effective amount of a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor, together with one or more pharmaceutically acceptable excipients or carriers;
wherein compositions i) and ii) are separate compositions.

12. A drug combination as defined in claim 9, a single pharmaceutical composition as defined in claim 10, or a kit of parts as defined in claim 11, for use in the prophylactic or the therapeutic treatment of adverse ventricular remodelling and of heart failure.

13. The LAT1 selective inhibitor or a pharmaceutically acceptable salt thereof, or any stereoisomer or mixtures of stereoisomers of any of them, for use in the prophylactic or the therapeutic treatment of adverse ventricular remodelling and of heart failure, wherein the compound is to be administered simultaneously, concurrently, separately or sequentially with a therapeutic agent selected from the group consisting of an angiotensin-converting enzyme (ACE) inhibitor, a angiotensin receptor neprilysin inhibitor (ARNi), a beta-adrenergic receptor inhibitor, a mineralocorticoid receptor antagonist, and a sodium-glucose cotransporter type 2 (SGLT2) inhibitor.

14. The LAT1 selective inhibitor for use according to claim 1, wherein the LAT1 selective inhibitor is for a sustained or prolonged use.

15. The LAT1 selective inhibitor for use according to claim 14, wherein the sustained or prolonged use on a once daily administration, particularly, for the rest of the subject life.
